(19) Europäisches Patentamt
European Patent Office
Office européen des brevets

(11) **EP 3 768 391 B1**

(12) **FASCICULE DE BREVET EUROPEEN**

(45) Date de publication et mention de la délivrance du brevet:
**05.01.2022 Bulletin 2022/01**

(51) Int Cl.:
*A61Q 19/00* (2006.01)    *A61Q 19/08* (2006.01)
*A61K 8/9783* (2017.01)

(21) Numéro de dépôt: **19718781.8**

(86) Numéro de dépôt international:
**PCT/FR2019/050618**

(22) Date de dépôt: **19.03.2019**

(87) Numéro de publication internationale:
**WO 2019/180368 (26.09.2019 Gazette 2019/39)**

(54) **COMPOSITION COSMÉTIQUE COMPRENANT UNE HUILE ESSENTIELLE D'IMMORTELLE ET UN EXTRAIT HUILEUX DE DRÈCHES D'IMMORTELLE**

KOSMETISCHE ZUBEREITUNG ENTHALTEND EIN ÄTHERISCHES IMMORTELLENÖL UND EINEN EXTRAKT AUS IMMORTELLETREBER

COSMETIC COMPOSITION COMPRISING AN ESSENTIAL OIL OF EVERLASTING AND AN EXTRACT OF EVERLASTING'S SPENT GRAINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.03.2018 FR 1852359**

(43) Date de publication de la demande:
**27.01.2021 Bulletin 2021/04**

(73) Titulaire: **LABORATOIRES M & L**
**04100 Manosque (FR)**

(72) Inventeurs:
• CENIZO, Valérie
13650 Meyrargues (FR)
• COMBES, Charline
04100 Manosque (FR)
• LEMAIRE, Géraldine
04220 Corbieres-en-Provence (FR)
• PORTES, Pascal
13540 Puyricard (FR)
• ROUQUET, Virginie
04100 Manosque (FR)

(74) Mandataire: **Cabinet Becker et Associés**
**25, rue Louis le Grand**
**75002 Paris (FR)**

(56) Documents cités:
FR-A1- 2 819 718    FR-A1- 2 975 006
FR-A1- 3 003 167

• Matthew Campbell: "Miraculous, Healing Helichrysum (Immortelle) Essential Oil Spotlightby", , 14 août 2017 (2017-08-14), XP002785345, Extrait de l'Internet: URL:https://www.katesmagik.com/blogs/news/miraculous-healing-helichrysum-immortelle-essential-oil [extrait le 2018-10-04]
• STELA JOKI,AMARINA RAJI,BBLANKA BILI AND MAJA MOLNAR: "Supercritical Extraction of Scopoletin fromHelichrysum italicum (Ro th) G. Don Flowers", PHYTOCHEM. ANAL., vol. 27, 2016, pages 290-295, XP002785346,

EP 3 768 391 B1

## Description

### DOMAINE DE L'INVENTION

La présente invention concerne une composition cosmétique renfermant une huile essentielle d'immortelle et un extrait huileux de drèches d'immortelle, ainsi que son utilisation cosmétique pour le soin de la peau, en particulier pour lutter contre les signes cutanés du vieillissement.

### ARRIERE-PLAN DE L'INVENTION

Les femmes et les hommes ont tendance actuellement à vouloir paraître jeunes le plus longtemps possible et cherchent par conséquent à estomper les marques du vieillissement de la peau, qui se traduisent notamment par des rides et des ridules.

La peau est constituée de deux compartiments, l'un superficiel, l'épiderme, et l'autre plus profond, le derme, qui interagissent. L'épiderme humain naturel est composé principalement de trois types de cellules qui sont les kératinocytes, très majoritaires, les mélanocytes, et les cellules de Langerhans. Chacun de ces types cellulaires contribue par ses fonctions propres au rôle essentiel joué dans l'organisme par la peau, notamment le rôle de protection de l'organisme des agressions extérieures appelé « fonction barrière ».

L'épiderme est conventionnellement divisé en une couche basale de kératinocytes constituant la couche germinative de l'épiderme, une couche dite épineuse constituée de plusieurs couches de cellules polyédriques disposées sur les couches germinatives, une à trois couches dites granuleuses constituées de cellules aplaties contenant des inclusions cytoplasmiques distinctes, les grains de kératohyaline, et enfin la couche cornée (ou stratum corneum), constituée d'un ensemble de couches de kératinocytes au stade terminal de leur différenciation et appelés cornéocytes. Il y a en permanence dans l'épiderme production de nouveaux kératinocytes pour compenser la perte en continu de cellules épidermiques au niveau de la couche cornée. L'homéostasie de la peau, et en particulier de l'épiderme, résulte d'une balance finement régulée entre les processus de prolifération et de différenciation des cellules de la peau. Ces processus de prolifération et de différenciation sont parfaitement régulés : ils participent au renouvellement et/ou à la régénération de la peau et conduisent au maintien d'une épaisseur constante de la peau, et en particulier d'une épaisseur constante de l'épiderme. L'homéostasie de la peau participe également au maintien des propriétés mécaniques de la peau.

La vitesse et la qualité de renouvellement de l'épiderme sont toutefois modifiées en permanence par les agressions physiques et chimiques auxquelles la peau est soumise, ainsi que par le vieillissement.

Ainsi, la vitalité cellulaire des kératinocytes peut être diminuée notamment dans le cadre du vieillissement ou à cause du stress oxydatif (par exemple rayonnement solaire i.e. UV, visible, infrarouge, lumière bleue), à cause de l'agression de l'épiderme par les toxines ou les métabolites de la microflore, ou, plus généralement, lors du vieillissement chronologique. Précisément, le potentiel régénératif de l'épiderme devient moins important, c'est-à-dire que les cellules de la couche basale se divisent moins activement, ce qui conduit à un ralentissement et/ou une diminution du renouvellement épidermique qui ne compense plus la perte des cellules éliminées en surface. Il en résulte une atrophie de l'épiderme et/ou une diminution de l'épaisseur de la peau. Les altérations de l'homéostasie épidermique se traduisent également par un aspect terne et/ou brouillé du teint. Une altération de la fonction barrière est également observée, qui se manifeste par différents signes selon la localisation : peau sèche, hyperkératose, épiderme fin, lèvres fines, rides superficielles.

L'altération de la barrière cutanée observée avec l'âge est en outre liée au relâchement de la cohésion cellulaire au niveau de l'épiderme, entraînant une fragilisation et une déshydratation de l'épiderme. Une augmentation de l'expression de protéines impliquées dans la différenciation et l'adhésion des kératinocytes favorise la formation et l'étanchéité de la barrière cutanée, ce qui permet, d'une part, de maintenir l'hydratation de la peau, et, d'autre part, de protéger la peau des agressions extérieures et de contrer son vieillissement. De tels gènes sont notamment les gènes codant pour la Transglutaminase 1 (TGM1), enzyme capable de catalyser la liaison entre les précurseurs de l'enveloppe cornée et la membrane cellulaire, et qui joue un rôle clé dans la différenciation épidermique.

On comprend donc l'intérêt de disposer d'une composition cosmétique permettant d'augmenter la différenciation épidermique sans affecter sensiblement le pool de cellules basales, afin de retarder l'apparition des signes de vieillissement cutané ou de ralentir leur progression et également de prévenir ou traiter le dessèchement cutané.

La Demanderesse a déjà proposé d'utiliser une huile essentielle d'immortelle, riche en composés terpéniques, pour renforcer la fonction barrière (WO 2012/153064), en association avec un extrait aqueux et un extrait huileux d'immortelle. L'extrait huileux d'immortelle est obtenu par exemple par extraction aux micro-ondes et se caractérise par le fait qu'il renferme au moins 99% en poids d'acides gras et de 0,2 à 0,5% en poids de phytostérols.

De son côté, le document FR 2 819 718 décrit un extrait lipidique de plante, notamment d'immortelle, obtenu par extraction au $CO_2$ supercritique, en présence d'un solvant composé de triglycérides d'origine végétale (triglycérides caprylique / caprique). Après élimination du solvant et ajout d'un composé cireux, il a été démontré que cet extrait lipidique agissait sur la différenciation cellulaire à la manière de l'acide rétinoïque, en augmentant l'expression du gène CRABP-II codant pour la protéine cellulaire liant l'acide rétinoïque, ainsi que pour le facteur de croissance endothélial (VEGF), et que cet extrait stimulait la division (prolifération) des kératinocytes. En revanche, il est souligné que l'extrait lipidique inhibe (-61%) l'expression de gènes codant pour les protéines habituellement exprimées au cours de la différenciation épidermique, telles que la transglutaminase 1 (TGM1).

RESUME DE L'INVENTION

Dans ce contexte, la Demanderesse a mis en évidence de manière tout-à-fait surprenante une stimulation, de manière synergique, des gènes régulant la différenciation épidermique lorsque l'huile essentielle d'immortelle était associée à un extrait huileux d'immortelle obtenu spécifiquement à partir de drèches d'immortelle, notamment une augmentation de l'expression du gène codant pour la protéine TGM1.

Plus précisément, la Demanderesse a démontré que la combinaison d'un extrait huileux de drèches d'immortelle avec une huile essentielle d'immortelle conduisait à une augmentation synergique de l'expression des marqueurs de différenciation que constituent la transglutaminase 1, la cytokératine 10, l'involucrine, la filaggrine, la cathepsine L2 et la loricrine. Il est ainsi apparu à la Demanderesse que la combinaison précitée permettait de formuler une composition cosmétique efficace contre les signes du vieillissement cutané et/ou le dessèchement de la peau.

Dans ce contexte, l'invention a pour objet une composition cosmétique comprenant une huile essentielle d'immortelle et un extrait huileux de drèches d'immortelle, lequel extrait huileux de drèches d'immortelle renferme des stérols ; des acides carboxyliques linéaires, saturés ou insaturés, en $C_7$-$C_{10}$, y compris l'acide 2-(Z)-décénoïque et l'acide 4-(Z)-décénoïque ; de l'acide benzoïque ; et des esters éthyliques d'acides gras linéaires, saturés ou insaturés, en $C_8$-$C_{18}$.

Elle a encore pour objet l'utilisation non thérapeutique de la composition précitée pour ralentir l'apparition, réduire la progression ou diminuer l'intensité des signes du vieillissement cutané, notamment les rides, le relâchement cutané, la perte de souplesse et/ou d'élasticité de la peau, l'amincissement de la peau, la rugosité de la peau et/ou la perte d'éclat du teint ; ou pour prévenir ou traiter le dessèchement de la peau.

L'invention a également pour objet un procédé cosmétique pour ralentir l'apparition, réduire la progression ou diminuer l'intensité des signes du vieillissement cutané, notamment la formation de rides, le relâchement cutané, la perte de souplesse et/ou d'élasticité de la peau, l'amincissement de la peau, la rugosité de la peau et/ou la perte d'éclat du teint, ou pour prévenir ou traiter le dessèchement de la peau, comprenant l'application topique sur la peau de la composition précitée.

DESCRIPTION DETAILLEE

La présente invention met en œuvre une huile essentielle d'immortelle et un extrait huileux de drèches d'immortelle.

Parmi les espèces d'immortelle susceptibles d'être indépendamment utilisées pour préparer l'huile essentielle et l'extrait huileux de drèches, on citera toutes celles relevant du genre *Helichrysum* et en particulier *Helichrysum italicum* (ou *Helichrysum angustifolium* D.C), qui est l'immortelle d'Italie, *Helichrysum arenarium,* qui est l'immortelle des sables, et *Helichrysum stoechas* ou immortelle commune, sans que cette liste ne soit limitative. On utilise préférentiellement une huile essentielle d'*Helichrysum italicum,* quelle que soit la sous-espèce considérée.

Par "huile essentielle", on entend dans cette description le produit d'hydrodistillation ou d'entraînement à la vapeur d'eau des composés organiques volatils présents dans une partie quelconque de l'immortelle ou de la plante entière, et plus particulièrement dans ses parties aériennes comme par exemple ses fleurs ou sommités fleuries. Dans ces procédés, les molécules odorantes contenues dans la plante sont libérées et entraînées mécaniquement avec la vapeur d'eau qui est soit formée par ébullition de l'eau à laquelle la plante est ajoutée (hydrodistillation classique), soit formée par ébullition de l'eau contenue dans la plante (hydrodistillation par micro-ondes, éventuellement sous vide), soit encore fournie par une chaudière et mise en circulation à travers la plante (entraînement à la vapeur d'eau). Le distillat est ensuite décanté pour séparer l'huile essentielle utilisée selon l'invention de l'hydrolat (ou eau florale). Le résidu solide qui constitue les drèches peut être récupéré et traité pour préparer l'extrait huileux de drèches d'immortelle également utilisé dans la présente invention.

L'huile essentielle d'immortelle représente avantageusement de 0,001 à 5% en poids et préférentiellement de 0,001 à 1% en poids, par rapport au poids total de la composition selon l'invention.

Elle est associée, dans cette composition, à un extrait huileux de drèches d'immortelle. Par "drèches", on entend le résidu solide restant après hydrodistillation ou entraînement à la vapeur d'eau de toute partie de l'immortelle et séparation de l'huile essentielle et de l'hydrolat produits. L'extrait huileux de drèches peut être obtenu par tout procédé d'extraction d'huile et plus particulièrement par extraction des drèches à l'aide d'un fluide supercritique, tel que le dioxyde de carbone, le monoxyde d'azote, le diméthyléther, le propane, l'éthylène ou le méthanol, sans que cette liste ne soit limitative. On préfère utiliser le dioxyde de carbone à l'état supercritique. L'homme du métier saura ajuster les paramètres d'extraction et notamment la température, la pression et le débit du fluide en fonction de la vitesse d'extraction et du rendement voulus. Par exemple, l'extraction à l'aide de $CO_2$ supercritique peut être effectuée à une pression de 250 à 300 bars et à une température de 40 à 80°C, de préférence de 50 à 70°C. Après extraction, le fluide supercritique est ramené à l'état gazeux par décompression et généralement recyclé. L'extrait végétal ainsi obtenu est avantageusement ensuite soumis à une étape de distillation moléculaire, par exemple à une température de 180 à 250°C, de préférence de 200 à 220°C, afin de concentrer les insaponifiables présents dans l'extrait. Avant cette étape de distillation moléculaire, l'extrait végétal peut être éventuellement dilué dans un solvant huileux, notamment à base d'huile végétale telle que de l'huile de tournesol. Ce solvant huileux est éliminé lors de l'étape de distillation moléculaire. On obtient ainsi un distillat renfermant notamment de 30 à 50% en poids, plus particulièrement de 35 à 45% en poids, d'insaponifiables et de 1 à 10% en poids, plus particulièrement de 3 à 7% en poids, de stérols, essentiellement du beta-sitostérol.

L'extrait obtenu à l'aide du fluide supercritique ou le distillat résultant de l'étape de distillation moléculaire peut éventuellement être dilué dans un solvant, à une concentration de 1 à 10% en poids, par exemple, notamment de 3 à 5% en poids, afin de faciliter sa manipulation. Tous les solvants huileux utilisables en cosmétique peuvent être utilisés à cette fin, notamment les triglycérides d'acides gras, tels que le triglycéride caprylique / caprique.

La Demanderesse a caractérisé l'extrait huileux de drèches d'immortelle par chromatographie en phase gazeuse couplée à la spectrométrie de masse. Il a ainsi été démontré que l'extrait huileux de drèches d'immortelle utilisé selon l'invention se différenciait, sur le plan de sa composition, d'un extrait obtenu par $CO_2$ supercritique de sommités fleuries d'immortelle par le fait qu'il ne renfermait sensiblement pas (c'est-à-dire moins de 10% en poids, voire moins de 5% en poids ou même moins de 2% en poids), voire pas du tout, d'acide palmitoléique, de linalol, de 2-décénal, de 2-tridécanone, d'heptadécane, de 8-heptadécène, d'acide isobutyrique, d'acide isovalérique, d'hexadécénol, d'acide laurique, d'acide pentadécanoïque, d'acide décanoïque et d'acide isostéarique. A contrario, l'extrait huileux de drèches d'immortelle utilisé selon l'invention contient des constituants qui n'ont pas été identifiés dans un extrait obtenu par $CO_2$ supercritique de sommités fleuries d'immortelle, à savoir les esters méthyliques d'acide 2(Z)-décénoïque et d'acide 4(Z)-décénoïque. En outre, par comparaison avec une huile essentielle d'immortelle, l'extrait huileux de drèches d'immortelle utilisé selon l'invention renferme des stérols, essentiellement du beta-sitostérol, des esters méthyliques d'acides carboxyliques linéaires, saturés ou insaturés, en $C_7$-$C_{10}$ (en particulier des acides heptanoïque, octénoïque et décénoïque), l'ester méthylique de l'acide benzoïque et des esters éthyliques d'acides gras linéaires, saturés ou insaturés, en $C_8$-$C_{18}$ (en particulier caprylate, caprate et linoléate).

Un tel extrait est notamment identifié sous le nom INCI : HELICHRYSUM ITALICUM FLOWER STEM EXTRACT.

L'extrait huileux de drèches d'immortelle représente avantageusement de 0,001 à 5% en poids et préférentiellement de 0,001 à 1% en poids, par rapport au poids total de la composition selon l'invention.

Comme il ressort des exemples ci-après, il a été démontré que la combinaison d'extraits de plantes selon l'invention permet d'activer de manière synergique l'expression des gènes impliqués dans la régulation de la différenciation épidermique.

Par "synergie", on entend dans le cadre de cette description que l'effet obtenu en utilisant la combinaison d'extraits selon l'invention est significativement supérieur à l'effet obtenu en utilisant l'un quelconque des deux extraits et éventuellement supérieur à la somme des effets produits par chacun des extraits.

La combinaison précitée est incluse dans une composition cosmétique qui renferme un milieu physiologiquement acceptable, en particulier cosmétiquement acceptable, c'est-à-dire qui ne génère pas de picotements ou de rougeurs incompatibles avec une utilisation cosmétique. Ce milieu renferme de préférence une phase aqueuse et une phase grasse (contenant généralement l'huile essentielle d'immortelle et l'extrait huileux de drèches d'immortelle). On préfère que la composition se présente sous forme d'émulsion huile-dans-eau ou eau-dans-huile ou d'une dispersion d'huile dans l'eau.

La phase aqueuse renferme de l'eau et éventuellement au moins un constituant choisi parmi les polyols et les gélifiants aqueux. L'eau représente avantageusement de 40 à 80%, par exemple de 50 à 70%, du poids total de la composition. Le polyol peut notamment être choisi parmi la glycérine, le propylène glycol, le butylène glycol, le pentylène glycol et leurs mélanges et il peut représenter de 5 à 30%, de préférence de 15 à 25%, du poids total de la composition.

Par "gélifiant aqueux", on désigne un composé polymérique capable d'immobiliser des molécules d'eau en s'hydratant et d'augmenter ainsi la viscosité de la phase aqueuse. Un tel gélifiant peut être choisi parmi : les polysaccharides, tels que : la cellulose et ses dérivés, les amidons modifiés, le carraghénane, l'agar agar, la gomme de xanthane et les gommes végétales telles que la gomme de guar ou de caroube ; les polymères synthétiques et notamment les homopolymères d'acrylate de sodium éventuellement réticulés, ainsi que les copolymères acryliques, en particulier les copolymères d'acrylate de sodium et/ou de (méth)acrylate d'alkyle et/ou de (méth)acrylate d'hydroxyalkyle et/ou de (méth)acrylate de (polyéthoxy)alkyle, avec éventuellement au moins un autre monomère, avantageusement l'acide 2-acrylamido-2-méthylpropane sulfonique (AMPS), ces copolymères étant éventuellement réticulés ; et leurs mélanges.

De son côté, la phase grasse peut comprendre une ou plusieurs huiles volatiles et/ou non volatiles. Des exemples d'huiles volatiles sont les alcanes ramifiés, tels que l'isododécane, et les alcanes linéaires en $C_{10}$-$C_{13}$. Comme huiles non volatiles, on peut citer notamment :

- les esters d'acides et de mono-alcool choisis parmi : les mono- et polyesters d'acides linéaires saturés en C2-C10 (de préférence en C6-C10) et de mono-alcools linéaires saturés en C10-C18 (de préférence C10-C14), les mono- et polyesters d'acides linéaires saturés en C10-C20 et de mono-alcools ramifiés ou insaturés en C3-C20 (de préférence C3-C10) ; les mono- et polyesters d'acides ramifiés ou insaturés en C5-C20 et de mono-alcools ramifiés ou insaturés en C5-C20 ; les mono- et polyesters d'acides ramifiés ou insaturés en C5-C20 et de mono-alcools linéaires en C2-C4 ;
- les triglycérides d'acides gras en C6-C12, tels que les triglycérides d'acides caprylique et caprique et la triheptanoïne ;
- les acides gras ramifiés et/ou insaturés en C10-C20 (tels que les acides linoléique, laurique et myristique) ;
- les alcools gras ramifiés et/ou insaturés en C10-C20 (tels que l'octyldodécanol et l'alcool oléylique) ;
- les hydrocarbures tels que le squalane (C30), notamment le squalane végétal extrait de l'huile d'olive, et l'hémisqualane (C15) ;
- les carbonates de dialkyle, tels que le dicaprylyl carbonate et le diéthylhexyl carbonate ;
- les dialkyléthers tels que le dicaprylyl éther ; et
- leurs mélanges.

On peut également citer les huiles végétales qui contiennent un ou plusieurs des constituants précités.

Comme esters d'acides et de monoalcools, on peut notamment citer les monoesters tels que le mélange de caprate et caprylate de coco, le macadamiate d'éthyle, l'ester éthylique de beurre de karité, l'isostéarate d'isostéaryle, l'isononanoate d'isononyle, l'isononanoate d'éthylhexyle, le néopentanoate d'hexyle, le néopentanoate d'éthylhexyle, le néopentanoate d'isostéaryle, le néopentanoate d'isodécyle, le myristate d'isopropyle, le myristate d'octyldodécyle, le palmitate d'isopropyle, le palmitate d'éthylhexyle, le laurate d'hexyle, le laurate d'isoamyle, le nonanoate de cétostéaryle, le capylate de propylheptyle et leurs mélanges. D'autres esters utilisables sont les diesters d'acides et de monoalcools tels que l'adipate de disopropyle, l'adipate de diéthylhexyle, le sébaçate de diisopropyle et le sébaçate de diisoamyle. Des exemples d'huiles végétales sont notamment les huiles de germe de blé, de tournesol, d'argan, d'hibiscus, de coriandre, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de cassis, d'onagre, de lavande, de bourrache, de millet, d'orge, de quinoa, de seigle, de carthame, de bancoulier, de passiflore, de rosier muscat, d'Echium, de cameline ou de camélia.

La phase grasse peut en outre comprendre au moins un structurant de phase grasse. Par "structurant de phase grasse", on entend un composé capable d'épaissir les huiles contenues dans la composition, choisi notamment parmi les cires, les gélifiants de phase grasse et les corps gras pâteux, ainsi que leurs mélanges.

Selon une forme d'exécution préférée, cette composition renferme en outre au moins un actif anti-âge autre qu'un extrait d'immortelle, en particulier un actif adapté à prévenir et/ou traiter les rides, le relâchement cutané et/ou la formation de taches pigmentaires, qui peut notamment être choisi parmi les agents anti-radicalaires, les agents stimulant la différenciation et/ou la prolifération des kératinocytes et/ou des fibroblastes ; les agents stimulant la synthèse de glycosaminoglycanes et/ou de collagène et/ou de fibrilles d'ancrage dermo-épidermique et/ou des fibres élastiques; les agents prévenant la dégradation du collagène et/ou des glycosaminoglycanes et/ou des fibrilles d'ancrage dermo-épidermique et/ou des fibres élastiques ; les agents antiglycation ; les agents dépigmentants et/ou inhibant la mélanogénèse ; et leurs mélanges.

Des exemples de tels actifs anti-âge sont notamment : l'acide ascorbique, ses sels, ses éthers et ses esters, notamment le glucoside d'ascorbyle ; l'adénosine ; le ribose ; les extraits de miel ; les protéines et glycoprotéines, extraites notamment d'amande douce ; les protéines végétales hydrolysées, notamment issues du riz, des graines d'hibiscus ou du lupin ; les polypeptides et les pseudodipeptides, tels que le chlorhydrate de carcinine, le palmitoyl pentapeptide-4 (Pal-Lys-Thr-Thr-Lys-Ser) et le palmitoyl tripeptide-38 commercialisés notamment par SEDERMA sous les dénominations commerciale Matrixyl® 3000 et Matrixyl® Synthe'6, respectivement, le palmitoyl tripeptide-8 commercialisé par la société LUCAS MEYER sous la référence commerciale Nutrazen®, le pentapeptide-18 commercialisé par la société LIPOTEC sous la dénomination commerciale Leuphasyl® Solution, le sh-decapeptide-9 commercialisé par la société SANDREAM sous la dénomination commerciale Neoendorphin® et le palmitoyl hexapeptide-52 commercialisé par la société INFINITEC sous la référence commerciale X50 Myocept® Powder ; les silanes tels que le mannuronate de méthylsilanol ; les arabinoxylanes, extraits en particulier de farine de seigle et les galactoarabinanes, issus notamment du mélèze ; l'acide hyaluronique et ses sels ; les polyphénols, extraits en particulier de mimosa ; les alpha-hydroxyacides, dont ceux extraits de citron; les extraits (généralement aqueux) de plantes telles que le trèfle d'eau, la pensée sauvage, la prêle des champs, la Mafane (*Acmella oleracea*), le chardon aux ânes (*Onopordum acanthium*), le millefeuille (*Achillea millefolium*, contenu notamment dans le produit Neurobiox® de la société BASF), l'embelia (*Embelia concinna*, telle que commercialisée par la société SEPPIC), le figuier de Barbarie (*Opuntia ficus indica*, commercialisé notamment par MIBELLE AG BIOCHEMISTRY sous la dénomination commerciale AquaCacteen®), la sauge (*Salvia officinalis*, vendue notamment par PROVITAL GROUP), *Vitex negundo* (commercialisé notamment par les LABORATOIRES EXPANSCIENCE sous la référence commerciale Neurovity®), la châtaigne, la papaye, l'arganier, l'avoine, le tournesol, la pâquerette, la pivoine ou l'aneth ; les extraits aqueux d'algues et notamment de coralline, de janie rouge, d'*Ungaria pinnatifada*, d'*Alaria esculenta* ou de *Nannochlorosis oculata* ; les huiles essentielles, notamment de myrte ; les gluconates de zinc et/ou de cuivre ; et leurs mélanges.

En variante ou en plus, la composition utilisée selon l'invention peut comprendre au moins un agent tenseur. Il peut s'agir d'un polymère tenseur, capable de tendre la peau par action mécanique et de réduire ainsi l'apparence des rides et des ridules, en particulier d'un polysaccharide, notamment d'un extrait d'algue ou de plancton marin ou d'une gomme végétale. Il peut s'agir également d'un agent tenseur agissant par voie biologique du type « botox-like », par exemple, un extrait d'*Acmella oleracea* commercialisé sous le nom de Gatuline® Expression par la société GATTEFOSSE ; un extrait de graines d'hibiscus commercialisé sous le nom de Myoxinol® LS9736 par la société BASF BCS ou encore un peptide de type Acétyl Hexapeptide-8 commercialisé sous le nom d'Argireline® par la société Lipotec.

La composition selon l'invention peut en outre contenir différents constituants qui peuvent être dispersés dans la phase grasse et/ou dans la phase aqueuse de l'émulsion, pour autant que ceux-ci soient compatibles avec une application topique sur la peau.

Elle peut ainsi renfermer au moins un émulsionnant huile-dans-eau ou eau-dans-huile, généralement non ionique, tel que des esters de polyoxyéthylène, des esters de sorbitane éventuellement polyéthoxylés, des esters d'acides gras et de glycérol éventuellement polyéthoxylés, des éthers d'alcools gras et de sucre tels que les alkyl glucosides, et leurs mélanges. Les émulsionnants peuvent représenter de 2 à 10% et de préférence de 4 à 6% du poids total de la composition.

La composition selon l'invention peut également comprendre une ou plusieurs charges pulvérulentes, qui se présentent avantageusement sous forme de microparticules poreuses ou creuses, de préférence poreuses. Ces microparticules sont en principe sensiblement sphériques. Ces charges peuvent notamment être choisies parmi :

- les charges organiques telles que : les poudres de polysaccharides et en particulier d'amidon natif, d'amidon modifié ou de cellulose ; les poudres de polymères acryliques tels que le poly(méthacrylate de méthyle), de polyamides ou de polyoléfines ; les poudres d'algues séchées telles que *Corallina officinalis* ;
- les charges inorganiques telles que la silice, les argiles, la perlite et le talc ;
- et leurs mélanges.

Comme charge inorganique, on préfère utiliser la silice.

Ces charges peuvent représenter de 1 à 5% en poids, par rapport au poids total de la composition.

La composition selon l'invention peut en outre comprendre des additifs choisis notamment parmi des agents photoprotecteurs organiques et/ou inorganiques, actifs dans la lumière bleue et/ou l'UVA et/ou l'UVB ; des polymères filmogènes à base de polysaccharides, capables de former un film protecteur anti-pollution, tels que les produits commercialisés par SOLABIA sous les dénominations commerciales Pollustop® et Solashield® ; des agents desquamants tels que des α- et β-hydroxyacides ; des particules exfoliantes ; des parfums ; des agents anti-oxydants ; des agents séquestrants ; des ajusteurs de pH ; des conservateurs ; des pigments ; des colorants ; et leurs mélanges.

Cette composition peut se présenter sous toute forme adaptée à une application topique sur la peau et notamment sous forme de lait, de crème, de lotion, de gel, de pâte ou de film. Il s'agit généralement d'une composition non rincée et en particulier d'une composition de soin, d'une composition de maquillage, telle qu'un fond de teint, ou d'une composition solaire.

La composition selon l'invention peut être appliquée sur au moins une zone du corps d'une personne présentant des signes de vieillissement et/ou dessèchement cutané (par exemple d'au moins 30 ans, voire d'au moins 40 ans, et plus particulièrement sur le visage, le cou et/ou le décolleté. En variante, elle peut être appliquée sur les mains. En variante encore, elle peut être appliquée sur l'ensemble du corps, notamment sur le buste, les bras, les jambes et le ventre, en vue de lutter contre les signes de vieillissement et/ou de dessèchement de la peau.

Cette composition peut être appliquée une ou plusieurs fois par jour, par exemple matin et/ou soir, sur les zones à traiter.

En variante, la composition selon l'invention peut être une composition rincée utilisée pour le soin de la peau, en particulier du visage et éventuellement du corps. Dans ce cas, elle peut par exemple être utilisée comme masque ou comme pâte de gommage.

FIGURES

La Figure 1 illustre le taux d'expression des gènes codant pour la transglutaminase 1 (TGM1), la cytokératine 10 (KRT10) et l'involucrine (IVL), exprimés par des kératinocytes en culture traités avec la composition selon l'invention, par rapport à un témoin non traité.

La Figure 2 illustre le taux d'expression des gènes codant pour la filaggrine (FLG), la cathepsine 2 (CSTL2) et la loricrine (LOR), exprimés par des kératinocytes en culture traités avec la composition selon l'invention, par rapport à un témoin non traité.

La Figure 3 illustre le taux d'expression des gènes codant pour l'antigène identifié par l'anticorps monoclonal Ki-67 (MKI67), l'intégrine β1 (ITGB1) et l'intégrine α6 (ITGA6), exprimés par des kératinocytes en culture traités avec la composition selon l'invention, par rapport à un témoin non traité.

EXEMPLES

L'invention sera mieux comprise à la lumière des exemples suivants, qui sont donnés à titre purement illustratif et n'ont pas pour but de limiter la portée de l'invention, définie par les revendications annexées.

**Exemple 1** : **Test** *in vitro* **sur culture primaire de kératinocytes humains : Effet sur la différenciation épidermique de l'association selon l'invention**

1. Matériels & Méthode

On a utilisé dans les essais ci-après une huile essentielle d'*Helichrysum italicum* obtenue par entrainement à la vapeur des sommités fleuries de la plante et un extrait huileux de drèches d'immortelle obtenu à partir du résidu d'entrainement à la vapeur. Cet extrait a été préparé par extraction du résidu (drèches) au $CO_2$ supercritique à une température de 60 °C et sous une pression de 285 bars. Les insaponifiables ainsi obtenus ont été dilués dans l'huile de tournesol, puis soumis à une étape de distillation moléculaire à 200°C et le distillat résultant a été standardisé en le diluant à hauteur de 3-5% en poids dans des triglycérides caprylique / caprique.

*a) Culture cellulaire*

Des kératinocytes en culture primaire, provenant d'un donneur féminin d'une biopsie mammaire, ont été ensemencés dans des plaques 96 puits (Nunc) à raison de 12000 cellules par puit dans le milieu Keratinocyte Serum-Free Medium (KSFM, Gibco) supplémenté avec 50 µg/ml d'extrait de glande pituitaire (Gibco), 5 mg/ml EGF (Epidermal Growth Factor, Gibco) et 100 µg/ml de normocine (Invivogen). Les cellules ont été maintenues 3 jours en culture à 37°C et 5% de $CO_2$ avant d'être traitées par l'extrait huileux de drèches d'immortelle, l'huile essentielle d'immortelle et leur association..

*b) Cytotoxicité de l'huile essentielle d'immortelle, de l'extrait huileux de drèches d'immortelle et de leur association*

Après 72h de culture, le milieu a été changé, en présence ou non de l'huile essentielle d'immortelle à 0,0005%, de l'extrait huileux de drèches d'immortelle à 0,001% et de leur association. Pour chaque concentration, une culture a été réalisée sur 6 puits identiques.

La culture a été maintenue pendant 4 jours et le milieu contenant les composés de l'étude a été changé 3 fois (chaque jour) au cours du temps de culture.

Après 4 jours de culture, le milieu de culture a été éliminé et remplacé par un milieu contenant une solution de XTT (sel de sodium de 2,3-bis(2-méthoxy-4-nitro-5-sulfophényl)-5-[(phénylamino)-carbonyl]-2H-tétrazolium). Les cellules ont été incubées avec cette solution pendant 2h à 37°C et 5% de $CO_2$.

A la fin de la période d'incubation, l'absorbance a été mesurée à 450 nm à l'aide d'un lecteur de microplaques Varioskan®.

La viabilité cellulaire a été calculée de la manière suivante :

$$\% \text{ viabilité} = (\text{moyenne des DO traitement} - \text{blanc}) / (\text{moyenne des DO sans traitement} - \text{blanc}) \times 100$$

Le seuil de toxicité a été fixé à 85% de la valeur du contrôle sans traitement.

*c) Extraction des ARN*

Après avoir mesuré l'absorbance pour la viabilité cellulaire, le milieu contenant la solution de XTT a été éliminé et remplacé par un tampon (Buffer RLT, Qiagen) contenant 1% de β-mercaptoéthanol (VWR). Les cellules, après avoir été lysées, ont été congelées à -80°C. Afin d'avoir assez d'ARN pour réaliser les analyses de PCR en temps réel, 3 puits ont été poolés par condition et 2 extractions distinctes d'ARNs ont été réalisées selon le protocole du RNeasy mini kit (Qiagen) avec une incubation à la DNase I pour éliminer l'ADN génomique. La quantité des ARNs a été mesurée à l'aide d'un spectrophotomètre NanoView et la qualité des ARNs a été vérifiée par électrophorèse en micro-capillaire à l'aide du Bioanalyzer 2100 (Agilent Technologies).

*d) Rétrotranscription-PCR*

400 ng d'ARNs ont été rétro-transcrits en ADNc à l'aide du kit «AffinityScript QPCR cDNA synthesis kit» (Agilent technologies). Les PCR en temps réel ont été réalisées en duplicata pour chaque condition à l'aide du système MXP 3005 (Agilent technologies). Le kit d'amplification utilisé était Brilliant III Ultra-Fast SYBR Green QPCR Master Mix (Agilent technologies)

Les amorces utilisées provenaient de chez Bio-Rad : transglutaminase 1 (TGM1, qHsaCED0056475), kératine 10 (KRT10, qHsaCED0034304), involucrine (IVL, qHsaCED0046054), filaggrine (FLG, qHsaCED0036604), cathepsine 2 (CSTL2, qHsaCED0044736), loricrine (LOR, qHsaCED0048415), intégrine β1 (ITGB1, qHsaCED0005248) et intégrine α6 (ITGA6, qHsaCED0042632) 2 gènes de ménages *TATA box binding protein* (TBP, qHsaCID0007122) et *ribosomal protein L13a* (RPL13A, qHsaCED0045063) ont été utilisés pour normaliser l'expression du gène d'intérêt.

## 2. Résultats

Il a été démontré que les extraits testés séparément, ainsi que leur mélange, ne présentaient pas de cytotoxicité (viabilité cellulaire > 85%).

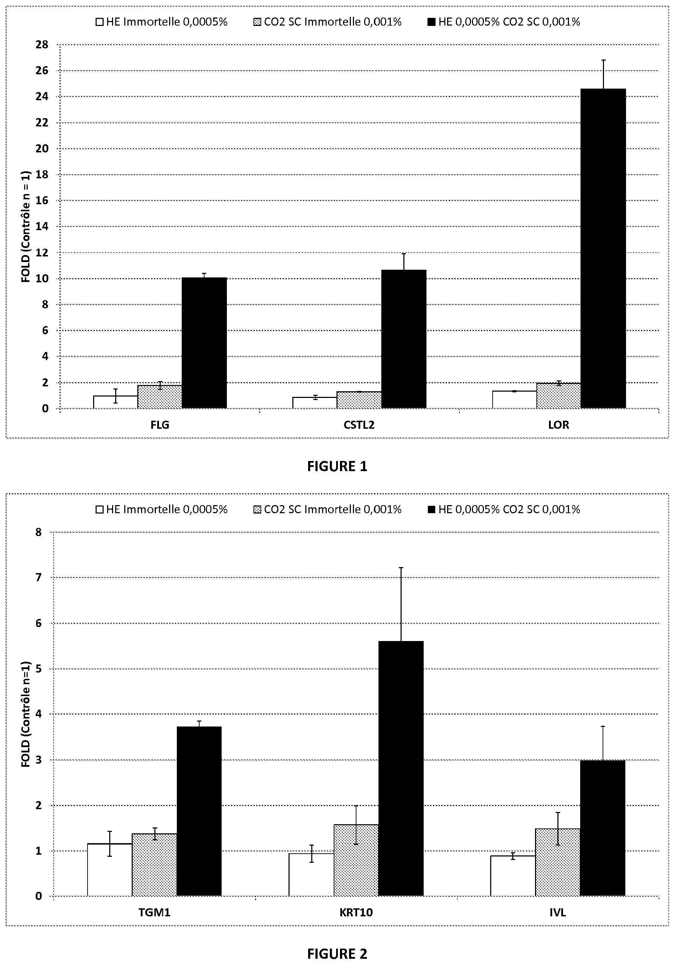

Par ailleurs, il a été mis en évidence (Figures 1 et 2) que l'expression des gènes impliqués dans la différenciation épidermique et la fonction barrière (TGM1, KRT10, IVL, FLG, CSTL2 et LOR) est augmentée de manière synergique en utilisant la combinaison des deux extraits (huile essentielle d'immortelle et extrait huileux de drèches d'immortelle), par rapport à chaque extrait pris séparément. L'expression de ces gènes est multipliée par un facteur de 3 à 24 avec cette combinaison, alors que ces gènes ne sont pas sensiblement surexprimés par chacun des extraits. Cet effet est particulièrement prononcé pour le gène codant pour la loricrine, et dans une moindre mesure pour le gène codant pour l'involucrine, qui sont respectivement des protéines insoluble et soluble essentielles à la formation de l'enveloppe cornée et participant à la fonction barrière de l'épiderme, reliées l'une à l'autre par TGM1 qui est une enzyme calcium-dépendante. On observe également une surexpression très forte du gène codant pour la filaggrine, qui constitue un ciment entre les filaments de kératine constituant également la couche cornée et précurseur du facteur naturel d'hydratation (NMF).

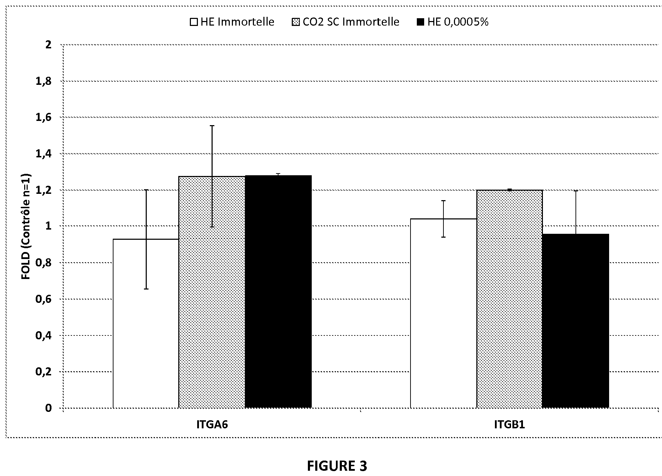

Par ailleurs, comme il ressort de la Figure 3, on n'observe pas de modification de l'expression des marqueurs des kératinocytes indifférenciés (ITGA6 et ITGB1) ce qui suggère notamment que la combinaison selon l'invention préserve un pool constant de cellules prolifératives, assurant ainsi le renouvellement épidermique .

Cet exemple démontre ainsi que la combinaison selon l'invention permet de lutter efficacement contre le vieillissement de la peau et de renforcer la fonction barrière de la peau.

## Exemple 2 : Composition cosmétique

La composition suivante est préparée de manière classique pour l'homme de l'art, en mélangeant les ingrédients ci-dessous dans les proportions pondérales indiquées.

| | |
|---|---|
| Huile essentielle d'immortelle | 0,1 % |
| Extrait huileux de drèches d'immortelle | 0,1 % |
| Adénosine | 0,04 % |
| Huiles | 5-15 % |
| Gélifiants huileux | 1-5 % |
| Gélifiants aqueux | 1-3 % |
| Polyols | 20-25 % |
| Séquestrant | qs |
| Anti-oxydants | qs |
| Parfum | qs |

(suite)

| Conservateurs | | qs |
| Eau | qsp | 100 % |

## Revendications

**1.** Composition cosmétique comprenant, dans un milieu physiologiquement acceptable, une huile essentielle d'immortelle et un extrait huileux de drèches d'immortelle, lequel extrait huileux de drèches d'immortelle renferme des stérols ; des acides carboxyliques linéaires, saturés ou insaturés, en $C_7$-$C_{10}$, y compris l'acide 2-(Z)-décénoïque et l'acide 4-(Z)-décénoïque ; de l'acide benzoïque ; et des esters éthyliques d'acides gras linéaires, saturés ou insaturés, en $C_8$-$C_{18}$.

**2.** Composition selon la revendication 1, **caractérisée en ce que** l'immortelle utilisée dans la préparation de l'huile essentielle et de l'extrait huileux est indépendamment choisie parmi les espèces *Helichrysum italicum, Helichrysum arenarium* et *Helichrysum stoechas,* de préférence *Helichrysum italicum.*

**3.** Composition selon la revendication 1 ou 2, **caractérisée en ce que** l'huile essentielle d'immortelle est obtenue à partir des parties aériennes de la plante, en particulier de ses fleurs ou sommités fleuries.

**4.** Composition selon l'une quelconque des revendications 1 à 3, **caractérisée en ce que** l'extrait huileux de drèches d'immortelle est obtenu par extraction des drèches à l'aide d'un fluide supercritique, de préférence le dioxyde de carbone à l'état supercritique, l'extrait obtenu étant ensuite éventuellement soumis à une étape de distillation moléculaire.

**5.** Composition selon l'une quelconque des revendications 1 à 4, **caractérisée en ce que** l'extrait huileux de drèches d'immortelle est dilué dans un solvant tel que les triglycérides d'acide caprylique / caprique.

**6.** Composition selon l'une quelconque des revendications 1 à 5, **caractérisée en ce que** l'huile essentielle d'immortelle représente de 0,001 à 5% en poids et préférentiellement de 0,001 à 1% en poids, par rapport au poids total de la composition.

**7.** Composition selon l'une quelconque des revendications 1 à 6, **caractérisée en ce que** les stérols sont essentiellement constitués du beta-sitostérol, les acides carboxyliques linéaires, saturés ou insaturés, en $C_7$-$C_{10}$ comprennent les acides heptanoïque, octénoïque et décénoïque et les esters éthyliques d'acides gras linéaires, saturés ou insaturés, en $C_8$-$C_{18}$ comprennent les caprylate, caprate, et linoléate d'éthyle.

**8.** Composition selon l'une quelconque des revendications 1 à 7, **caractérisée en ce que** l'extrait huileux de drèches d'immortelle représente de 0,001 à 5% en poids et préférentiellement de 0,001 à 1% en poids, par rapport au poids total de la composition.

**9.** Utilisation non thérapeutique de la composition selon l'une quelconque des revendications 1 à 8 pour ralentir l'apparition, réduire la progression ou diminuer l'intensité des signes du vieillissement cutané, notamment les rides, le relâchement cutané, la perte de souplesse et/ou d'élasticité de la peau, l'amincissement de la peau, la rugosité de la peau et/ou la perte d'éclat du teint ; ou pour prévenir ou traiter le dessèchement de la peau.

**10.** Procédé cosmétique pour ralentir l'apparition, réduire la progression ou diminuer l'intensité des signes du vieillissement cutané, notamment la formation de rides, le relâchement cutané, la perte de souplesse et/ou d'élasticité de la peau, l'amincissement de la peau, la rugosité de la peau et/ou la perte d'éclat du teint, ou pour prévenir ou traiter le dessèchement de la peau, comprenant l'application topique sur la peau de la composition selon l'une quelconque des revendications 1 à 8.

## Patentansprüche

**1.** Kosmetische Zusammensetzung, enthaltend in einem physiologisch verträglichen Medium ein ätherisches Immortelle-Öl und einen öligen Extrakt aus Immortelle-Treber, wobei der ölige Extrakt aus Immortelle-Treber Sterole;

lineare, gesättigte oder ungesättigte $C_7$-$C_{10}$-Carbonsäuren, einschließlich 2-(Z)-Decensäure und 4-(Z)-Decensäure; Benzoesäure; und Ethylester linearer, gesättigter oder ungesättigter $C_8$-$C_{18}$-Fettsäuren enthält.

2. Zusammensetzung nach Anspruch 1, **dadurch gekennzeichnet, dass** die bei der Herstellung des ätherischen Öls und des öligen Extrakts verwendete Immortelle unabhängig ausgewählt ist aus den Arten *Helichrysum italicum, Helichrysum arenarium* und *Helichrysum stoechas,* vorzugsweise *Helichrysum italicum.*

3. Zusammensetzung nach Anspruch 1 oder 2, **dadurch gekennzeichnet, dass** das ätherische Immortelle-Öl aus den oberirdischen Teilen der Pflanze, insbesondere deren Blüten oder Blütenspitzen gewonnen wird.

4. Zusammensetzung nach einem der Ansprüche 1 bis 3, **dadurch gekennzeichnet, dass** der ölige Extrakt aus Immortelle-Treber durch Extraktion der Treber unter Verwendung eines überkritischen Fluids, vorzugsweise Kohlendioxid im überkritischen Zustand, erhalten wird, wobei der erhaltene Extrakt dann gegebenenfalls einem molekularen Destillationsschritt unterzogen wird.

5. Zusammensetzung nach einem der Ansprüche 1 bis 4, **dadurch gekennzeichnet, dass** der ölige Extrakt aus Immortelle-Treber in einem Lösungsmittel wie Triglyceriden von Capryl-/Caprinsäure verdünnt ist.

6. Zusammensetzung nach einem der Ansprüche 1 bis 5, **dadurch gekennzeichnet, dass** das ätherische Immortelle-Öl 0,001 bis 5 Gew.-% und vorzugsweise 0,001 bis 1 Gew.-%, bezogen auf das Gesamtgewicht der Zusammensetzung, ausmacht.

7. Zusammensetzung nach einem der Ansprüche 1 bis 6, **dadurch gekennzeichnet, dass** die Sterole im Wesentlichen aus Beta-Sitosterol bestehen, die linearen, gesättigten oder ungesättigten $C_7$-$C_{10}$-Carbonsäuren Heptan-, Octen- und Decensäuren umfassen, und die Ethylester linearer, gesättigter oder ungesättigter $C_8$-$C_{18}$-Fettsäuren Ethyl-Caprylat, -Caprat und -Linoleat umfassen.

8. Zusammensetzung nach einem der Ansprüche 1 bis 7, **dadurch gekennzeichnet, dass** der ölige Extrakt aus Immortelle-Treber 0,001 bis 5 Gew.-% und vorzugsweise 0,001 bis 1 Gew.-%, bezogen auf das Gesamtgewicht der Zusammensetzung, ausmacht.

9. Nicht-therapeutische Verwendung der Zusammensetzung nach einem der Ansprüche 1 bis 8 zur Verlangsamung des Einsetzens, Verringern des Fortschreitens oder Vermindern der Intensität der Zeichen der Hautalterung, insbesondere der Falten, der Haut-Erschlaffung, dem Verlust der Flexibilität und/oder der Elastizität der Haut, der Ausdünnung der Haut, der Rauhigkeit der Haut und/oder der Verlust der Ausstrahlung des Teints; oder zur Verhinderung oder Behandlung der Trockenheit der Haut,.

10. Kosmetisches Verfahren zur Verlangsamung des Einsetzens, Verringern des Fortschreitens oder Vermindern der Intensität der Zeichen der Hautalterung, insbesondere der Bildung von Falten, der Haut-Erschlaffung, dem Verlust der Flexibilität und/oder der Elastizität der Haut, der Ausdünnung der Haut, der Rauhigkeit der Haut und/oder der Verlust der Ausstrahlung des Teints; oder zur Verhinderung oder Behandlung der Trockenheit der Haut, umfassend die topische Anwendung der Zusammensetzung nach einem der Ansprüche 1 bis 8 auf der Haut.

## Claims

1. A cosmetic composition comprising, in a physiologically acceptable medium, an essential oil of immortelle and an oily extract of immortelle distillation residue, which oily extract of immortelle distillation residue contains sterols; saturated or unsaturated, linear $C_7$-$C_{10}$ carboxylic acids, including 2-(Z)-decenoic acid and 4-(Z)-decenoic acid; benzoic acid; and ethyl esters of saturated or unsaturated, linear $C_8$-$C_{18}$ fatty acids.

2. The composition as claimed in claim 1, **characterized in that** the immortelle used in the preparation of the essential oil and of the oily extract is independently chosen from the species *Helichrysum italicum, Helichrysum arenarium* and *Helichrysum stoechas,* preferably *Helichrysum italicum.*

3. The composition as claimed in claim 1 or 2, **characterized in that** the essential oil of immortelle is obtained from the aerial parts of the plant, in particular from its flowers or flowering heads.

4. The composition as claimed in any one of claims 1 to 3, **characterized in that** the oily extract of immortelle distillation residue is obtained by extraction of the distillation residue using a supercritical fluid, preferably carbon dioxide in the supercritical state, the extract obtained then being optionally subjected to a molecular distillation step.

5. The composition as claimed in any one of claims 1 to 4, **characterized in that** the oily extract of immortelle distillation residue is diluted in a solvent such as caprylic/capric acid triglycerides.

6. The composition as claimed in any one of claims 1 to 5, **characterized in that** the essential oil of immortelle represents from 0.001 to 5% by weight and preferentially from 0.001 to 1% by weight, relative to the total weight of the composition.

7. The composition as claimed in any one of claims 1 to 6, **characterized in that** the sterols essentially consist of beta-sitosterol, the saturated or unsaturated, linear $C_7$-$C_{10}$ carboxylic acids comprise heptanoic, octenoic and decenoic acids and the ethyl esters of saturated or unsaturated, linear $C_8$-$C_{18}$ fatty acids comprise ethyl caprylate, ethyl caprate and ethyl linoleate.

8. The composition as claimed in any one of claims 1 to 7, **characterized in that** the oily extract of immortelle distillation residue represents from 0.001 to 5% by weight and preferentially from 0.001 to 1% by weight, relative to the total weight of the composition.

9. The non-therapeutic use of the composition as claimed in any one of claims 1 to 8, for delaying the onset, reducing the progression or decreasing the intensity of the signs of skin aging, in particular wrinkles, sagging of the skin, loss of suppleness and/or of elasticity of the skin, thinning of the skin, roughness of the skin and/or loss of radiance of the complexion; or for preventing or treating dry skin.

10. A cosmetic process for delaying the onset, reducing the progression or decreasing the intensity of the signs of skin aging, in particular the formation of wrinkles, sagging of the skin, loss of suppleness and/or of elasticity of the skin, thinning of the skin, roughness of the skin and/or loss of radiance of the complexion, or for preventing or treating dry skin, comprising the topical application onto skin of the composition as claimed in any one of claims 1 to 8.

FIGURE 1

FIGURE 2

**FIGURE 3**

## RÉFÉRENCES CITÉES DANS LA DESCRIPTION

**Documents brevets cités dans la description**

- WO 2012153064 A
- FR 2819718